(19) Europäisches Patentamt / European Patent Office / Office européen des brevets

(11) **EP 4 781 971 A1**

(12) **EUROPEAN PATENT APPLICATION**
published in accordance with Art. 153(4) EPC

(43) Date of publication:
29.07.2026 Bulletin 2026/31

(21) Application number: 24894280.7

(22) Date of filing: 27.06.2024

(51) International Patent Classification (IPC):
*A61K 8/02* (2006.01)   *A61K 8/368* (2006.01)
*A61K 8/38* (2006.01)   *A61K 8/81* (2006.01)
*A61K 8/73* (2006.01)   *A61K 8/87* (2006.01)

(52) Cooperative Patent Classification (CPC):
A61K 8/368; A61K 8/0208; A61K 8/365;
A61K 8/38; A61K 8/73; A61K 8/731; A61Q 19/00;
A61Q 19/08; A61Q 19/10; B32B 7/06; B32B 27/08;
B32B 27/18; B32B 27/302; B32B 27/304;
B32B 27/306;                                    (Cont.)

(86) International application number:
PCT/KR2024/009010

(87) International publication number:
WO 2025/110383 (30.05.2025 Gazette 2025/22)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 24.11.2023 KR 20230166100

(71) Applicant: Skin Solution Co., Ltd
Ansan-si, Gyeonggi-do 15422 (KR)

(72) Inventor: NA, Yoo Sik
Suwon-si Gyeonggi-do 16514 (KR)

(74) Representative: Müller-Boré & Partner
Patentanwälte PartG mbB
Friedenheimer Brücke 21
80639 München (DE)

(54) **HYDROCOLLOID SHEET HAVING EXFOLIATING FUNCTION**

(57) Proposed is a hydrocolloid sheet with exfoliation performance, the hydrocolloid sheet including a release layer, a hydrocolloid layer stacked on an upper surface of the release layer and including a pressure-sensitive adhesive, an absorbent, and an exfoliant, and a base layer staked on an upper surface of the hydrocolloid layer and protecting the hydrocolloid layer.

FIG. 1

EP 4 781 971 A1

(52) Cooperative Patent Classification (CPC): (Cont.)
**B32B 27/32; B32B 27/36; B32B 27/40;**
**B32B 37/24;** A61K 2800/28; B32B 2037/243;
B32B 2250/02; B32B 2250/24; B32B 2255/10;
B32B 2255/24; B32B 2255/28; B32B 2307/726;
B32B 2307/7376; B32B 2307/748; B32B 2555/00

## Description

### Technical Field

The present disclosure relates to a hydrocolloid sheet with exfoliation performance.

### Background Art

Dead skin cells cover the skin of the body and serve as a barrier that prevents evaporation of moisture from the skin and entry of external foreign substances into the body. However, when excessive dead skin cells accumulate on the skin surface, they cause rough skin texture and dull skin tone. In addition, they impede absorption of skincare nutrients into the skin.

When dead skin cells and keratin plugs are left on the skin surface, they clog pores, leading to various problems such as acne disease, and dead cells and irregular melanin pigmentation in the stratum corneum of the skin cause aesthetic problems. These dead skin cells and keratin plugs cannot be removed even when the face is washed thoroughly, and even commonly used facial cleansers are not sufficient to completely remove the dead skin cells deep within pores. In addition, when the skin does not retain a certain level of moisture and becomes dry, it can lead to itching, and in severe cases, is prone to forming fine cracks. In this case, harmful bacteria can enter the skin through the cracks and cause skin diseases, so it is necessary to manage dead skin cells.

Conventional methods for removing dead skin cells or keratin plugs from the skin surface include a method of removing keratin plugs using cationic polymers and a method of removing keratin plugs using a sheet-type cosmetic composition composed of a film-forming cosmetic layer and a moisture-permeable substrate. However, despite of their effectiveness in removing dead skin cells and keratin plugs, these conventional methods are not only inconvenient to implement or use, but also problematic in that they cannot be carried around and remove unnecessary dead skin cells or keratin plugs from the skin surface at the desired time. Therefore, there is a need to develop a pressure-sensitive adhesive sheet for removing dead skin cells and keratin plugs that can effectively and easily remove unnecessary dead skin cells and keratin plugs from the skin surface without causing a burden on the skin.

The foregoing is intended merely to aid in the understanding of the background of the present disclosure, and is not intended to mean that the present disclosure falls within the purview of the related art that is already known to those skilled in the art.

[Documents of Related Art]

(Patent Document 1) Korean Patent Application Publication No. 10-2011-0113904 A (Publication Date: October 19, 2011)

### Disclosure

### Technical Problem

Accordingly, the present disclosure has been made keeping in mind the above problems occurring in the related art, and an objective of the present disclosure is to provide a hydrocolloid sheet with exfoliation performance that can remove unnecessary dead skin cells and keratin plugs from the skin surface by simply attaching the sheet to the skin.

### Technical Solution

In order to achieve the above objective, according to one aspect of the present disclosure, there is provided a hydrocolloid sheet with exfoliation performance, the hydrocolloid sheet including: a release layer; a hydrocolloid layer stacked on an upper surface of the release layer and including a pressure-sensitive adhesive, an absorbent, and an exfoliant; and a base layer staked on an upper surface of the hydrocolloid layer and protecting the hydrocolloid layer.In addition, the hydrocolloid layer may include 20 to 40 parts by weight of the absorbent and 0.1 to 15 parts by weight of the exfoliant, with respect to 100 parts by weight of the pressure-sensitive adhesive.

In addition, the pressure-sensitive adhesive may be one or more selected from the group consisting of a styrene isoprene styrene block copolymer (SIS), a styrene isoprene styrene block copolymer (SI), a styrene ethylene butylene styrene block copolymer (SEBS), a styrene butadiene styrene block copolymer (SBS), and polyisobutylene (PIB).

In addition, the absorbent may be one or more selected from the group consisting of sodium carboxymethyl cellulose, cellulose gum, carbomer, xantham gum, gelatin, collagen, pectin, hyaluronic acid, guar gum, sodium alginate, agar, konjac, and arabic gum.

In addition, the exfoliant may be one or more selected from the group consisting of salicylic acid, benzoyl peroxide, glycolic acid, lactic acid, malic acid, citric acid, and tartaric acid.

In addition, the exfoliant may be salicylic acid or benzoyl peroxide.

In addition, the release layer may have a thickness of 50 to 150 $\mu$m.

In addition, the hydrocolloid layer may have a thickness of 100 to 600 $\mu$m.

In addition, the base layer may have a thickness of 10 to 50 $\mu$m.

In addition, the base layer may be one or more selected from the group consisting of polyolefin (PO), polyvinyl alcohol (PVA), polystyrene (PS), polyvinyl chloride (PVC), polyurethane (PU), and polyethylene terephthalate (PET).

According to another aspect of the present disclosure, there is provided a method of manufacturing a hydrocolloid sheet with exfoliation performance, the method including: (a) preparing a mixture by mixing 20 to 40 parts by weight of an absorbent and 0.1 to 15 parts by weight of an exfoliant with 100 parts by weight of a pressure-sensitive adhesive; (b) coating the mixture of step (a) on the release layer to form a hydrocolloid layer; and (c) forming a base layer on an upper surface of the hydrocolloid layer of step (b).

**Advantageous Effects**

According to the present disclosure, it is possible to provide a hydrocolloid sheet with exfoliation performance that can remove unnecessary dead skin cells and keratin plugs from the skin surface by simply attaching the sheet to the skin.

In addition, it is possible to provide a hydrocolloid sheet with exfoliation performance that has excellent adhesion to the skin.

In addition, it is possible to provide a hydrocolloid sheet with exfoliation performance that does not cause irritation to the skin.

**Description of Drawings**

The above and other objectives, features, and other advantages of the present disclosure will be more clearly understood from the following detailed description when taken in conjunction with the accompanying drawings, in which:

FIG. 1 is a view illustrating a hydrocolloid sheet with exfoliation performance according to an embodiment of the present disclosure;

FIG. 2 is a flowchart illustrating a method of manufacturing a hydrocolloid sheet with exfoliation performance according to another embodiment of the present disclosure;

FIG. 3 is a view illustrating the results of analyzing the dead skin cell improvement effect of sheets manufactured according to Examples and Comparative Examples; and

FIG. 4 is a view illustrating the results of analyzing the water absorption of sheets manufactured according to Examples and Comparative Examples.

**Mode for Invention**

In one embodiment, the present disclosure provides a hydrocolloid sheet with exfoliation performance, the hydrocolloid sheet including a release layer, a hydrocolloid layer stacked on an upper surface of the release layer and including a pressure-sensitive adhesive, an absorbent, and an exfoliant, and a base layer staked on an upper surface of the hydrocolloid layer and protecting the hydrocolloid layer.

Hereinbelow, exemplary embodiments of the present disclosure will be described in detail with reference to the accompanying drawings such that the present disclosure can be easily embodied by one of ordinary skill in the art to which the present disclosure belongs. Various changes to the following embodiments are possible and the scope of the present disclosure is not limited to the following embodiments. Wherever possible, the same reference numerals will be used throughout the drawings and the description to refer to the same or like elements or parts.

In one embodiment, the present disclosure provides a hydrocolloid sheet with exfoliation performance, the hydrocolloid sheet including a release layer 100, a hydrocolloid layer 200 stacked on an upper surface of the release layer 100 and including a pressure-sensitive adhesive, an absorbent, and an exfoliant, and a base layer 300 staked on an upper surface of the hydrocolloid layer 200 and protecting the hydrocolloid layer 200.

The release layer 100 is a support that supports the hydrocolloid layer 200, and at the serves to prevent entry of external contaminants and protects the hydrocolloid layer 200 from external shock.

The release layer 100 may be one or more selected from the group consisting of polyolefin (PO), polyvinyl alcohol (PVA), polystyrene (PS), polyvinyl chloride (PVC), polyurethane (PU), and polyethylene terephthalate (PET), and may specifically be PET.

The release layer 100 is in the form of a film, and may specifically be a film made of PET, and more specifically, may be a silicone release-treated PET film.

The manufacturing method of the release layer 100 is not limited, and commercially available PET film may be purchased and used.

In addition, the thickness of the release layer 100 is not particularly limited, but the average thickness of the release layer 100 may be 50 to 150 $\mu$m, specifically 80 to 120 $\mu$m, and more specifically 90 to 110 $\mu$m.

When the average thickness of the release layer 100 is less than 50 $\mu$m, its function of protecting the hydrocolloid layer 200 from external shock may be reduced, it has a poor appearance, and workability is reduced when forming the hydrocolloid layer 200 on the release layer 100. When the average thickness of the release layer 100 exceeds 150 $\mu$m, flexibility is reduced.

The hydrocolloid layer 200 may be formed by coating on the upper surface of the release layer 100. In addition, the hydrocolloid layer 200 may include a pressure-sensitive adhesive, an absorbent, and an exfoliant.

The pressure-sensitive adhesive may be one selected from the group consisting of a styrene isoprene styrene block copolymer (SIS), a styrene isoprene styrene block copolymer (SI), a styrene ethylene butylene styrene block copolymer (SEBS), a styrene butadiene styrene block copolymer (SBS), and polyisobutylene (PIB), and may specifically be SIS.

The absorbent may be one or more selected from the group consisting of sodium carboxymethyl cellulose (CMC), cellulose gum, carbomer, xantham gum, gelatin, collagen, pectin, hyaluronic acid, guar gum, sodium alginate, agar, konjac, and arabic gum, and may specifically be CMC.

The exfoliant may be one or more selected from the group consisting of salicylic acid, benzoyl peroxide, glycolic acid, lactic acid, malic acid, citric acid, and tartaric acid, and may specifically be salicylic acid or benzoyl peroxide.

The hydrocolloid layer 200 may include 20 to 40 parts by weight of the absorbent and 0.1 to 15 parts by weight of the exfoliant, with respect to 100 parts by weight of the pressure-sensitive adhesive.

For example, when salicylic acid is used as the exfoliant, the content of the exfoliant may be 1 to 2 parts by weight, and when benzoyl peroxide is used as the exfoliant, the content of the exfoliant may be 0.1 to 15 parts by weight.

When the content of the exfoliant exceeds the above range, the exfoliation effect may not be obtained or side effects such as skin trouble may occur.

When the content of the absorbent is less than 20, the effectiveness of hydrocolloid may be reduced due to low absorption of wound fluid. When the content of the absorbent exceeds 40 parts by weight, adhesion may be reduced. Therefore, the above range is preferable.

In addition, the hydrocolloid sheet can exhibit a further improved exfoliation effect when it includes the pressure-sensitive adhesive, absorbent, and exfoliant in the above-described range.

The pressure-sensitive adhesive may have a pressure-sensitive adhesive strength of 500 to 2,000 gf/25 mm.

When the pressure-sensitive adhesive strength is less than 500 gf/25 mm, the exfoliation effect and adhesion may decrease due to a reduction in pressure-sensitive adhesive performance. When the pressure-sensitive adhesive strength exceeds 2,000 gf/25 mm, the hydrocolloid sheet may cause skin damage when peeled off from the face.

The average thickness of the hydrocolloid layer 200 may be 100 to 600 $\mu$m, specifically 150 to 350 $\mu$m, and more specifically 300 $\mu$m.

When the average thickness of the hydrocolloid layer 200 is less than 100 $\mu$m, the exfoliation effect may be reduced. When the average thickness of the hydrocolloid layer 200 exceeds 600 $\mu$m, the overall thickness of the sheet may be increased, productivity may be reduced, and flexibility may be reduced.

The base layer 300 may be formed on the upper surface of the hydrocolloid layer 200 to serve to protect the hydrocolloid layer 200 from external contaminates or external shock and smooth out facial wrinkles.

The base layer 300 may be one or more selected from the group consisting of polyolefin (PO), polyvinyl alcohol (PVA), polystyrene (PS), polyvinyl chloride (PVC), polyurethane (PU), and polyethylene terephthalate (PET), and may specifically be PU.

For example, the hydrocolloid sheet may be a sheet in which the hydrocolloid layer 200 is formed between the release layer 100 made of PET and the base layer 300 made of PU.

The base layer 300 is in the form of a film, and may specifically be a silicone release-treated film.

The manufacturing method of the base layer 300 is not limited.

In addition, the thickness of the base layer 300 is not particularly limited, but the average thickness of the base layer 300 may be 10 to 50 $\mu$m, specifically 15 to 30 $\mu$m, and more specifically 20 $\mu$m.

When the average thickness of the base layer 300 is less than 10 $\mu$m, its function of protecting the hydrocolloid layer 200 may be reduced. When the average thickness of the base layer 300 exceeds 50 $\mu$m, flexibility may be reduced, resulting in poor wearing comfort.

In another embodiment, the present disclosure provides a method of manufacturing a hydrocolloid sheet with exfoliation performance, the method including (a) preparing a mixture by mixing 20 to 40 parts by weight of an absorbent and 0.1 to 15 parts by weight of an exfoliant with 100 parts by weight of a pressure-sensitive adhesive, (b) coating the mixture of step (a) on the release layer 100 to form a hydrocolloid layer 200, and (c) forming a base layer 300 on an upper surface of the hydrocolloid layer 200 of step (b).

Step (a) of preparing the mixture

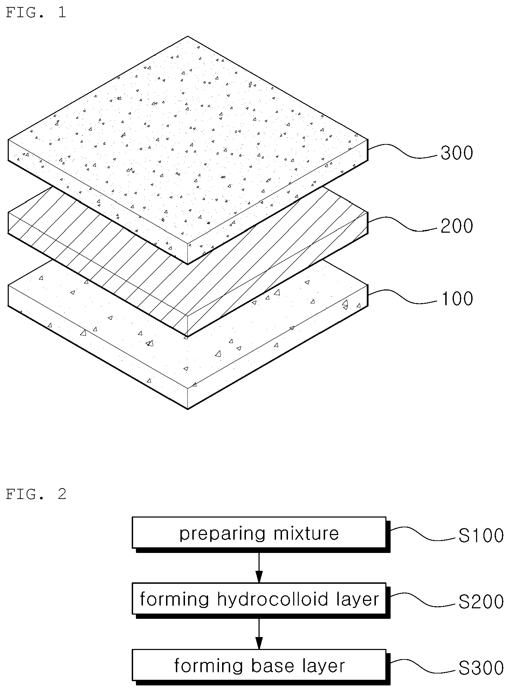

As illustrated in FIG. 2, a step S100 of preparing the mixture is performed.

In step (a), the mixture may be prepared by mixing 20 to 40 parts by weight of the absorbent and 0.1 to 15 parts by weight of the exfoliant with 100 parts by weight of the pressure-sensitive adhesive.

The mixing method is not limited as long as it can sufficiently mix all ingredients.

Step (b) forming the hydrocolloid layer

Next, as illustrated in FIG. 2, a step S200 of forming the hydrocolloid layer 200 is performed.

The mixture prepared in step (a) may be coated on the release layer 100 to form the hydrocolloid layer 200 on the upper surface of the release layer 100.

Here, the thickness of the release layer 100 is not particularly limited, but a release layer 100 having an average thickness of 50 to 150 $\mu$m, specifically 80 to 120 $\mu$m, and more specifically 90 to 110 $\mu$m may be used.

The release layer 100 may be a silicone release-treated film.

The method of coating the mixture on the release layer 100 is not limited, and the mixture may be coated so that the average thickness of the hydrocolloid layer 200 is 100 to 600 $\mu$m, specifically 150 to 350 $\mu$m, and more specifically 300 $\mu$m.

When the average thickness of the hydrocolloid layer 200 is less than 100 $\mu$m, the exfoliation effect may be reduced. When the average thickness of the hydrocolloid layer 200 exceeds 600 $\mu$m, the overall thickness of the sheet may be increased, productivity may be reduced, and flexibility may be reduced.

The release layer 100 may be one or more selected from the group consisting of polyolefin (PO), polyvinyl alcohol (PVA), polystyrene (PS), polyvinyl chloride (PVC), polyurethane (PU), and polyethylene terephthalate (PET), and may specifically be PET.

The manufacturing method of the release layer 100 is not limited.

Step (c) forming the base layer

Finally, as illustrated in FIG. 2, a step S300 of forming the base layer 300 is performed.

In step (c), the base layer 300 may be formed by stacking the base layer 300 in the form of a film on the hydrocolloid layer 200.

The base layer 300 and the hydrocolloid layer 200 may be easily bonded together by the pressure-sensitive adhesive included in the hydrocolloid layer 200.

Here, the base layer 300 may be a silicone release-treated film.

In addition, the thickness of the base layer 300 is not particularly limited, but a base layer 300 having an average thickness of 10 to 50 $\mu$m, specifically 15 to 30 $\mu$m, and more specifically 20 $\mu$m may be used.

Hereinafter, examples of the present disclosure will be described in more detail. These examples are merely for illustrative purposes, and it is obvious to those skilled in the art that the scope of the present disclosure is not limited thereto.

<Examples>

Example 1

A mixture was prepared by mixing 30 parts by weight of CMC and 2 parts by weight of salicylic acid with 100 parts by weight of SIS, and then the resulting mixture was coated to a thickness of 300 $\mu$m on an upper surface of a silicone release-treated PET film with a thickness of 100 $\mu$m to form a hydrocolloid layer. Thereafter, a PU film with a thickness of 20 $\mu$m was stacked on an upper surface of the hydrocolloid layer to manufacture a hydrocolloid sheet with exfoliation performance.

Example 2

A hydrocolloid sheet with improved exfoliation performance was prepared in the same manner as in Example 1, except that 10 parts by weight of benzoyl peroxide was used instead of salicylic acid.

<Comparative Examples>

**Comparative Example 1**

A hydrocolloid sheet was prepared in the same manner as in Example 1, except that salicylic acid included in a hydrocolloid layer was not used.

**Comparative Example 2**

A hydrocolloid sheet was prepared in the same manner as in Example 1, except that CMC included in a hydrocolloid layer was not used.

**<Test Examples>**

**Test Example 1: Analysis of dead skin cell improvement effect**

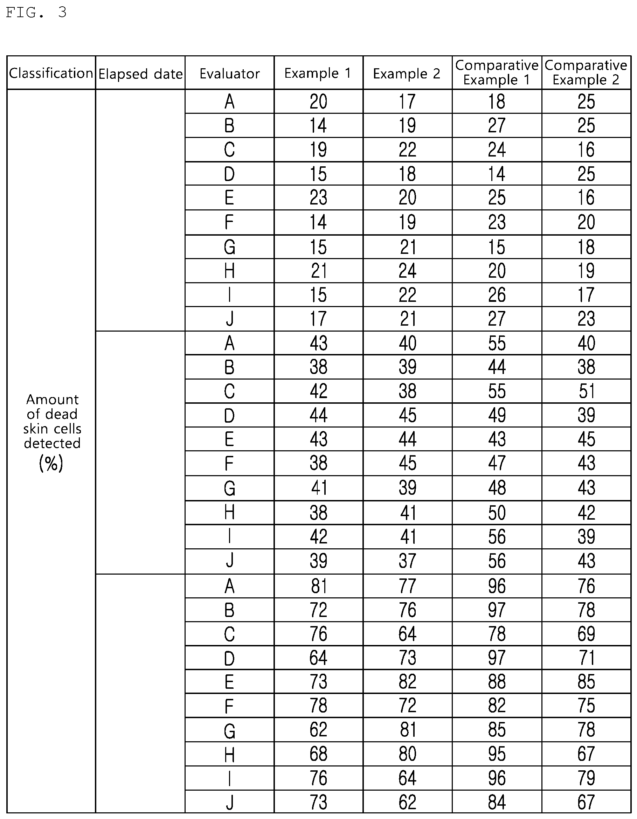

The dead skin cell improvement effect of the hydrocolloid sheets manufactured according to Examples 1 and 2 and Comparative Examples 1 and 2 was analyzed, and the results are illustrated in FIG. 3.
The test methods therefor are as follows.
10 evaluators aged 25 to 35 were asked to clean a test area (face) with a mild cleanser, wait for 20 minutes after 2 hours under measurement environmental conditions of a temperature of 22°C and a relative humidity of 50%, and expose the test area 10 minutes before measurement. Dead skin cells from the maxillary cheek area of the evaluators were collected using keratin tape. Samples of Examples and Comparative Examples were simultaneously attached to a location close to a keratin tape collection area and removed after 24 hours. Dead skin cells were collected from the corresponding location using keratin tape. After 3 and 7 days of evaluation, the evaluators washed his/her face with a mild cleanser and dead skin cells were collected from the same evaluation area again. The area of dead skin cells removed from the keratin tape was analyzed using Image-Pro Analyzer, and the dead skin cell improvement effect was expressed as the amount of dead skin cells detected (%).
Referring to FIG. 3, as a result of analyzing the amount of dead skin cells detected, it can be seen that the hydrocolloid sheets manufactured according to Examples 1 and 2 have a superior effect than those of Comparative Examples 1 and 2.

**Test Example 2: Evaluation of absorption**

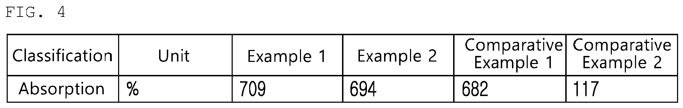

The absorption of the hydrocolloid sheets manufactured according to Examples 1 and 2 and Comparative Examples 1 and 2 was analyzed, and the results are illustrated in FIG. 4.
The absorption evaluation methods therefor are as follows.
Each sheet was cut to a size of 2 cm in width and length, weighed to measure an initial weight (W1), placed in distilled water at 37°C, and weighed to measure a weight (W2) after 48 hours.
The absorption (%) was calculated according to Equation 1 below.

**[Equation 1]**

$$\text{Absorption} = (W2/W1) \times 100$$

Referring to FIG. 4, it can be seen that the absorption of Comparative Example 1 excluding salicylic acid is lower than that of Examples 1 and 2, and in particular, the absorption of Comparative Example 2 excluding CMC is drastically lowered.
In other words, this indicates that the absorption is further improved when salicylic acid and CMC are used together.

**Industrial Applicability**

According to the present disclosure, there is provided a hydrocolloid sheet with exfoliation performance that can remove unnecessary dead skin cells and keratin plugs from the skin surface by simply attaching the sheet to the skin.

# EP 4 781 971 A1

**Claims**

1. A hydrocolloid sheet with exfoliation performance, the hydrocolloid sheet comprising:

   a release layer;
   a hydrocolloid layer stacked on an upper surface of the release layer and including a pressure-sensitive adhesive, an absorbent, and an exfoliant; and
   a base layer stacked on an upper surface of the hydrocolloid layer and protecting the hydrocolloid layer.

2. The hydrocolloid sheet of claim 1, wherein the hydrocolloid layer includes 20 to 40 parts by weight of the absorbent and 0.1 to 15 parts by weight of the exfoliant, with respect to 100 parts by weight of the pressure-sensitive adhesive.

3. The hydrocolloid sheet of claim 1, wherein the pressure-sensitive adhesive is one or more selected from the group consisting of a styrene isoprene styrene block copolymer (SIS), a styrene isoprene styrene block copolymer (SI), a styrene ethylene butylene styrene block copolymer (SEBS), a styrene butadiene styrene block copolymer (SBS), and polyisobutylene (PIB).

4. The hydrocolloid sheet of claim 1, wherein the absorbent is one or more selected from the group consisting of sodium carboxymethyl cellulose, cellulose gum, carbomer, xantham gum, gelatin, collagen, pectin, hyaluronic acid, guar gum, sodium alginate, agar, konjac, and arabic gum.

5. The hydrocolloid sheet of claim 1, wherein the exfoliant is one or more selected from the group consisting of salicylic acid, benzoyl peroxide, glycolic acid, lactic acid, malic acid, citric acid, and tartaric acid.

6. The hydrocolloid sheet of claim 1, wherein the exfoliant is salicylic acid or benzoyl peroxide.

7. The hydrocolloid sheet of claim 1, wherein the release layer has a thickness of 50 to 150 $\mu$m.

8. The hydrocolloid sheet of claim 1, wherein the hydrocolloid layer has a thickness of 100 to 600 $\mu$m.

9. The hydrocolloid sheet of claim 1, wherein the base layer has a thickness of 10 to 50 $\mu$m.

10. The hydrocolloid sheet of claim 1, wherein the base layer is one or more selected from the group consisting of polyolefin (PO), polyvinyl alcohol (PVA), polystyrene (PS), polyvinyl chloride (PVC), polyurethane (PU), and polyethylene terephthalate (PET).

11. A method of manufacturing a hydrocolloid sheet with exfoliation performance, the method comprising:

    (a) preparing a mixture by mixing 20 to 40 parts by weight of an absorbent and 0.1 to 15 parts by weight of an exfoliant with 100 parts by weight of a pressure-sensitive adhesive;
    (b) coating the mixture of step (a) on the release layer to form a hydrocolloid layer; and
    (c) forming a base layer on an upper surface of the hydrocolloid layer of step (b).

8

FIG. 1

300

200

100

FIG. 2

preparing mixture — S100

forming hydrocolloid layer — S200

forming base layer — S300

FIG. 3

| Classification | Elapsed date | Evaluator | Example 1 | Example 2 | Comparative Example 1 | Comparative Example 2 |
|---|---|---|---|---|---|---|
| Amount of dead skin cells detected (%) | | A | 20 | 17 | 18 | 25 |
| | | B | 14 | 19 | 27 | 25 |
| | | C | 19 | 22 | 24 | 16 |
| | | D | 15 | 18 | 14 | 25 |
| | | E | 23 | 20 | 25 | 16 |
| | | F | 14 | 19 | 23 | 20 |
| | | G | 15 | 21 | 15 | 18 |
| | | H | 21 | 24 | 20 | 19 |
| | | I | 15 | 22 | 26 | 17 |
| | | J | 17 | 21 | 27 | 23 |
| | | A | 43 | 40 | 55 | 40 |
| | | B | 38 | 39 | 44 | 38 |
| | | C | 42 | 38 | 55 | 51 |
| | | D | 44 | 45 | 49 | 39 |
| | | E | 43 | 44 | 43 | 45 |
| | | F | 38 | 45 | 47 | 43 |
| | | G | 41 | 39 | 48 | 43 |
| | | H | 38 | 41 | 50 | 42 |
| | | I | 42 | 41 | 56 | 39 |
| | | J | 39 | 37 | 56 | 43 |
| | | A | 81 | 77 | 96 | 76 |
| | | B | 72 | 76 | 97 | 78 |
| | | C | 76 | 64 | 78 | 69 |
| | | D | 64 | 73 | 97 | 71 |
| | | E | 73 | 82 | 88 | 85 |
| | | F | 78 | 72 | 82 | 75 |
| | | G | 62 | 81 | 85 | 78 |
| | | H | 68 | 80 | 95 | 67 |
| | | I | 76 | 64 | 96 | 79 |
| | | J | 73 | 62 | 84 | 67 |

FIG. 4

| Classification | Unit | Example 1 | Example 2 | Comparative Example 1 | Comparative Example 2 |
|---|---|---|---|---|---|
| Absorption | % | 709 | 694 | 682 | 117 |

## INTERNATIONAL SEARCH REPORT

| | International application No. |
| --- | --- |
| | **PCT/KR2024/009010** |

| A. | CLASSIFICATION OF SUBJECT MATTER |
| --- | --- |

**A61K 8/02**(2006.01)i; **A61K 8/368**(2006.01)i; **A61K 8/38**(2006.01)i; **A61K 8/81**(2006.01)i; **A61K 8/73**(2006.01)i; **A61K 8/87**(2006.01)i

According to International Patent Classification (IPC) or to both national classification and IPC

| B. | FIELDS SEARCHED |
| --- | --- |

Minimum documentation searched (classification system followed by classification symbols)

A61K 8/02(2006.01); A45D 44/00(2006.01); A61F 13/00(2006.01); A61F 13/02(2006.01); A61K 9/00(2006.01); A61K 9/70(2006.01); A61M 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 각질 제거제(exfoliating agent), 하이드로콜로이드(hydrocolloid), 시트(sheet)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2021-0099857 A (LEE, Ji Sang) 13 August 2021 (2021-08-13)<br>See abstract; claims 1 and 6-8; and paragraphs [0033], [0035] and [0037]. | 1-11 |
| Y | JP 2000-309522 A (L'OREAL SA) 07 November 2000 (2000-11-07)<br>See claims 1, 7-9, 13 and 15; and examples 1 and 2. | 1-11 |
| A | KR 10-2023-0098853 A (NATIONAL UNIVERSITY OF SINGAPORE) 04 July 2023 (2023-07-04)<br>See entire document. | 1-11 |
| A | KR 10-1995-0013505 A (AMOREPACIFIC CORPORATION) 15 June 1995 (1995-06-15)<br>See entire document. | 1-11 |
| A | KR 10-2001-0096776 A (KUNWOONG PHARM., CO., LTD.) 08 November 2001 (2001-11-08)<br>See entire document. | 1-11 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **30 September 2024** | **30 September 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 781 971 A1**

**INTERNATIONAL SEARCH REPORT**
Information on patent family members

| International application No. |
|---|
| **PCT/KR2024/009010** |

| Patent document cited in search report | | | Publication date (day/month/year) | Patent family member(s) | | | Publication date (day/month/year) |
|---|---|---|---|---|---|---|---|
| KR 10-2021-0099857 | A | | 13 August 2021 | None | | | |
| JP 2000-309522 | A | | 07 November 2000 | BR | 0001154 | A | 10 October 2000 |
| | | | | CA | 2301989 | A1 | 30 September 2000 |
| | | | | CN | 1156272 | C | 07 July 2004 |
| | | | | CN | 1273089 | A | 15 November 2000 |
| | | | | EP | 1043018 | A1 | 11 October 2000 |
| | | | | EP | 1043018 | B1 | 14 June 2006 |
| | | | | ES | 2265883 | T3 | 01 March 2007 |
| | | | | FR | 2791570 | A1 | 06 October 2000 |
| | | | | FR | 2791570 | B1 | 04 April 2003 |
| | | | | JP | 4224613 | B2 | 18 February 2009 |
| | | | | US | 2005-0191337 | A1 | 01 September 2005 |
| KR 10-2023-0098853 | A | | 04 July 2023 | CN | 116669592 | A | 29 August 2023 |
| | | | | EP | 4240202 | A1 | 13 September 2023 |
| | | | | US | 2023-0398040 | A1 | 14 December 2023 |
| | | | | WO | 2022-098314 | A1 | 12 May 2022 |
| KR 10-1995-0013505 | A | | 15 June 1995 | None | | | |
| KR 10-2001-0096776 | A | | 08 November 2001 | None | | | |

Form PCT/ISA/210 (patent family annex) (July 2022)

## EP 4 781 971 A1

**Patent documents cited in the description**

- KR 1020110113904A